(19) Europäisches Patentamt
European Patent Office
Office européen des brevets

(11) EP 0 548 344 B1

(12) EUROPEAN PATENT SPECIFICATION

(45) Date of publication and mention of the grant of the patent: **08.04.1998 Bulletin 1998/15**

(21) Application number: **92915903.6**

(22) Date of filing: **09.07.1992**

(51) Int. Cl.$^6$: **A61K 9/26**, A61K 9/00, A61K 9/70

(86) International application number: **PCT/US92/05606**

(87) International publication number: **WO 93/00890 (21.01.1993 Gazette 1993/03)**

(54) **COLLAGEN COMPOSITES FOR CONTROLLED DRUG RELEASE**

ZUSAMMENSETZUNGEN AUF DER BASIS VON COLLAGEN ZUR KONTROLLIERTEN ARZNEISTOFFREISETZUNG

COMPOSE DE LIBERATION CONTROLEE D'UN MEDICAMENT A BASE DE COLLAGENE

(84) Designated Contracting States: **AT BE CH DE DK ES FR GB GR IT LI LU MC NL SE**

(30) Priority: **09.07.1991 US 727508**

(43) Date of publication of application: **30.06.1993 Bulletin 1993/26**

(73) Proprietor: **VITAPHORE CORPORATION Menlo Park, CA 94025 (US)**

(72) Inventors:
- **YAMAMOTO, Ronald, K. Menlo Park, CA 94025 (US)**
- **BOND, Emmett, L. Mountain View, CA 94043 (US)**
- **DEVENS, Jennifer Oakland, CA 94619 (US)**
- **CHANG, Paul, Kan-Teh San Jose, CA 95129 (US)**

(74) Representative: **Cole, Paul Gilbert BERESFORD & Co 2-5 Warwick Court High Holborn London WC1R 5DJ (GB)**

(56) References cited:
**EP-A- 0 159 168**
**EP-A- 0 170 979**
**US-A- 2 824 092**
**US-A- 4 223 984**
**US-A- 4 865 846**
**US-A- 4 913 904**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.1/3.4

## Description

### FIELD OF THE INVENTION

The present invention is directed to collagen composites from which a drug may be loaded and released in a controlled manner. The present invention is particularly directed to collagen composites from which drugs having ophthalmic use may be released in a controlled manner.

### BACKGROUND OF THE INVENTION

In many drug delivery systems, involving percutaneous, subcutaneous or other in vivo application, a smooth release profile is desired to limit the adverse response to the drug or to prolong the duration of drug action. In particularly sensitive body tissues, such as the eye, careful control of the release of the drug is especially important and the type of substrate carrying the drug is somewhat limited since it must be transparent, thin, non-toxic, nonantigenic and nonimmunogenic as well as capable of bearing a sufficient load of the drug to be practically useful to combat the disorder or infection which is being treated.

Collagen, in the form of a thin film, gel, or microspheres, is a particularly attractive substrate for this purpose. However, the problem of adapting collagen materials for a sufficient load of drug and a smooth release profile sufficient for use within the eye has not been heretofore, it is believed, adequately solved.

One approach for altering the characteristics of collagen to attempt to tailor its drug release characteristics is disclosed in U.S. Patent 4,164,559 to Miyata et al. Collagen membranes are prepared wherein the collagen is modified by esterification and/or acylation, however, this chemical modification may unacceptably alter the collagen and create irritation and/or immunogenic reactions. Miyata et al. also describe proteolytic enzyme processing of collagen, however, this may also affect antigenicity and/or immunogenicity of the collagen.

In U.S. Patent 4,291,013 to Wahlig et al. there are disclosed collagen compositions from which active ingredient release may be attenuated by use of a resorbable binding agent. The preferred resorbable binding agents are polymers of glycolic acid and lactic acid, as well as their co-polymers in various weight ratios of the monomer units. Other disclosed resorbable binding agents are polyamino acids. The binding agent is used by mixing the binding agent, active ingredient and collagen by homogenization or other means and then pressing, sintering or melting the mixture. The shaping of the mixture is then carried out according to conventional synthetic resin processing technology under action of pressure and/or heat. The composition containing the active ingredient, binding agent and collagen is apparently fully resorbed into the body. It is not believed that the process described in this patent is applicable for making collagen composites wherein it is desired for the collagen to remain essentially intact while the drug is slowly released from the collagen in a controlled manner. It is believed that the invention described in this patent is not particularly useful for making collagen composites for ophthalmic use since it appears that the method of release of the drug is primarily by resorption of the entire composite, rather than by slow release of the drug from a substrate.

EP-A-0 170 979 (Merck) discloses a collagen-based delivery system in which a binding agent and active compound are added to collagen.

It is therefore an object of the present invention to provide collagen from which there is controlled release of a drug when in contact with body fluids or tissues, particularly when in contact with the eye.

This and other objects of the invention will be apparent from the following description, from the appended claims as well as from practice of the invention.

### SUMMARY OF THE INVENTION

The present invention provides a collagen-based drug delivery system having the features set out in claim 1 of the accompanying claims.

### BRIEF DESCRIPTION OF THE DRAWINGS

Fig. 1 is a graph of sequential uptake and delivery of collagen corneal shields unmodified or modified with ionic binding agents sodium alginate or polyquaternary ammonium cellulose, where the drug is simulated by the anionic marker sodium fluorescein.

Fig. 2 is a graph of the release of the drug gentamicin from collagen films modified with the ionic binding agent sodium alginate.

Fig. 3 is a graph of the release of the drug Gentamicin from collagen which has been modified with the ionic binding agent sodium alginate or quaternized cellulose.

Fig. 4 is a graph of the release rate of sodium fluorescein from collagen containing the ionic binding agent sodium

alginate or quaternized cellulose.

Fig. 5A is a graph of the uptake of dexamethasone as a function of weight of PEG - cholesteryl sebacate binding agent in a composite collagen film.

Fig. 5B is a graph of the bound fraction of dexamethasone as a function of wight of PEG - cholesteryl sebacate binding agent in a collagen film.

Fig. 6 is a graph of the uptake of cortisone as a function of weight of PEG - cholesteryl sebacate binding agent in a composite collagen film.

Fig. 7 is a graph of dexamethasone elution from a PEG - cholesteryl sebacate - modified composite collagen film.

Fig. 8A is a graph of a log y-axis plot of the data in Fig. 7, indicating the rate of elution of dexamethasone.

Fig. 8B is a graph of the half-life of release of dexamethasone from a PEG - cholesteryl sebacate-modified collagen film as a function of the bound fraction of dexamethasone in the film.

## DETAILED DESCRIPTION OF THE INVENTION

Non-antigenic, non-immunogenic substrates for delivery of drugs to body tissues and fluids are highly desirable particularly in sensitive areas, such as the eye. Collagen is an excellent material for delivery of drugs in that tissue since it readily hydrates for comfort and has a slow rate of dissolution in tears.

The collagen used in accordance with the present invention may comprise soluble or insoluble collagen of the types I, II, III and IV and mixtures thereof. Soluble collagens (types I, II, III and IV) may be prepared by known methods such as by enzymatic digestion of tissue enriched in such collagen types. Insoluble collagens may be derived from typical sources such as bovine and chicken tendons and bovine and chicken bones (all type I); bovine articular cartilage, nasal septum, sternal cartilage (all type II) and bovine and human aorta and skin (types I and III).

Methods of extraction and purification of various collagen material from such tissues are known and may vary somewhat, depending upon the nature of the collagen source. Tropocollagen or procollagen comprises the sodium chloride extract from collagen sources such as cartilage and bone. Mild acid extracts from collagen sources (such as by mild acetic acid extraction after removal of the more soluble substances through self-extraction) produces a greater yield of "acid soluble" collagen. Extraction may also be performed by limited cleavage by enzymes which preferentially degrade non-collagenous proteins (pepsin, papain, etc.).

The binding agents utilized in accordance with the present invention are believed to improve not only the loading but also the release of the drug from the collagen by tailoring the charge characteristics of the collagen-binding agent composite to complement the ionic charge, hydrophobicity and/or hydrophilicity of the bioactive agent. Unmodified collagen binds to both anionic and cationic bioactive agents due to the presence of both acidic and basic amino acid moieties in the collagen, however the binding agent improves the loading and release characteristics. In general, when dealing with a negatively charged or anionic bioactive agent, a positively charged or cationic binding agent should be utilized. When dealing with a cationic bioactive agent, then an anionic binding agent should be utilized.

Unmodified collagen binds to hydrophilic bioactive agents and to a lesser extent, to hydrophobic bioactive agents. However, an appropriate binding agent improves the loading and release characteristics of the bioactive agent. Particularly preferred are polymeric binding agents, such as, alginate and polyethyleneglycol derivatives which improve the loading and release characteristics in collagen of such drugs as tobramycin or dexamethasone (a steroid), respectively, as compared to unmodified collagen. In general, increasing hydrophobicity of the bioactive agent will require increase of the hydrophobicity of the binding agent to achieve appropriate loading and release profiles.

Examples of the binding agents are sodium alginate (a relatively hydrophilic polyanion available from kelp), carboxylated cellulose (a hydrophilic polyanion, derived from wood); polyacrylic acid and derivatives thereof (a hydrophilic polyanion which may be synthesized); glycosoaminoglycans (a hydrophilic polyanion, isolatable from animal tissues); polygalacturonic acid (a polyanion isolatable from citrus peels); pectin (a polyanion isolatable from citrus peels); carboxylated collagen (polyanions isolatable from animal tissues); dextran sulfate (polyanion, which may he synthesized); quaternized cellulose (polycations from chemically derivitized wood); polyethyleneglycol (a synthetic polymer) and derivatives thereof; spermine/spermidine (polycations isolatable from animals); chitin/chitosan (polycations isolatable from arthropods) and aminated collagen (polycation from chemically derivitized collagen isolatable from animal tissues).

Of particular advantage for hydrophobic bioactive agents is the use of a polyalkyleneglycol derivative of the bioactive agent as the binding additive. The coupling of polyethyleneglycol, for example, through a linking group, such as, sebacic acid, to a hydrophobic analogue of the hydrophobic bioactive agent renders the analogue water soluble. The resulting derivatives are miscible with collagen solutions and can be incorporated into collagen materials. The hydrophobic portion of the binding additive can provide specific hydrophobic binding attraction to the hydrophobic bioactive agent. A specific example is the use of polyethyleneglycol derivative of cholesterol, a biochemical precursor to steroids, as a binding agent to provide affinity for steroids such as dexamethasone:

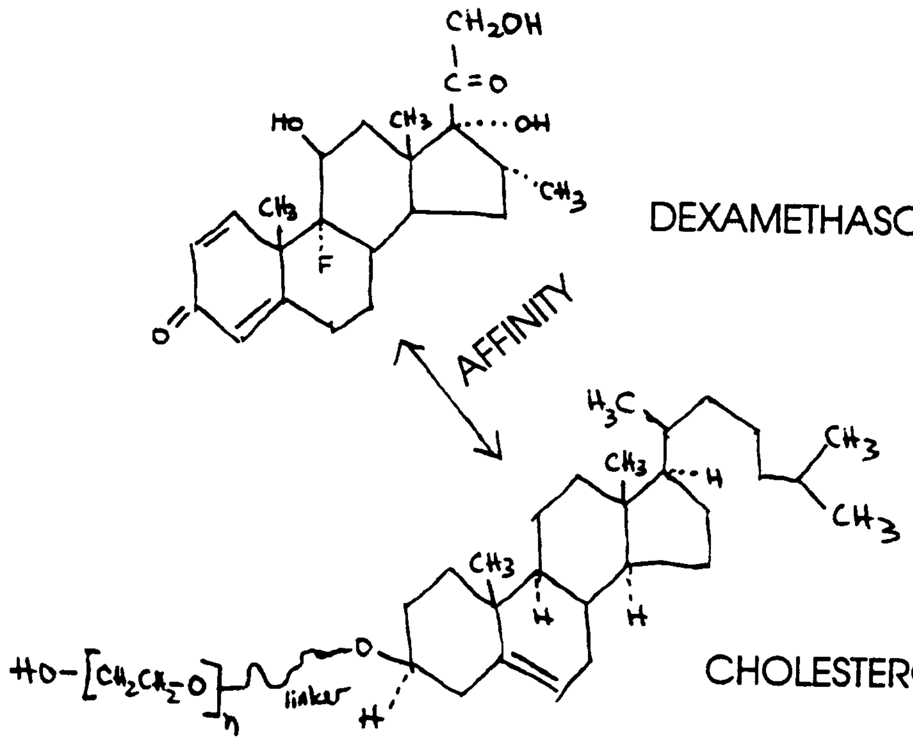


DEXAMETHASONE

CHOLESTEROL

polyethylene glycol

Methods of linking polyalkyleneglycols to other compounds are known in the art. For example, polyalkylene glycol may be linked to the hydroxy group of a sterol through a bifunctional linking agent which reacts with hydroxy groups, such as, diacids (succinic acid, glutaric acid, sebacic acid, etc.). Particularly preferred binding agents are polyethyleneglycol - and polypropyleneglycol-containing compounds.

A further improvement is attained in that the drug release rate is prolonged as compared to drug release from collagen, thus reducing the frequency of replacement of the drug delivery vehicle in the body. The polyalkyleneglycol-modified steroids include, but are not limited to, PEG-cholesterol derivatives, PEG-testosterone derivatives, PEG-estradiol derivatives, PEG-cortisone derivatives and the like.

The collagen and the binding agent are typically mixed in any convenient manner. A preferred method is to form an aqueous collagen dispersion, mix it with the binding agent, cast a dispersion into an appropriate mold, and then dry. The relative amounts of the collagen to binding agent will vary but in general about 2% by weight of the binding agent to 20% by weight may be utilized to retain the essential physical characteristics of the collagen and yet to impart sufficient appropriate ionic and/or hydrophobic or hydrophilic character to the collagen to obtain a desired load and release rate.

Other methods may be utilized to physically mix the collagen with the binding agent, such as by formulating dry mixtures of collagen and binding agent, then forming an aqueous suspension thereof.

The collagen-binding agent composites may then be treated to crosslink the collagen by conventional methods such as by dehydration, ultraviolet irradiation, by use of chemical crosslinking agents such as a carbodiimide, N-hydroxysuccinimide derived from active esters, etc. Examples include succinimide active esters such as N-hydroxysuccinimide, 3,3'-dithio (sulfosuccinimidyl propionate and bis- (sulfosuccinimidyl) suberate. In some instances, the binding agent may be covalently linked directly to the collagen, particularly if the molecular weight and size of the binding agent is such that collagen crosslinking is not sufficient to prevent loss of the binding agent when the composite is in an environment for releasing or for re-loading of the active agent from or into the composite.

Crosslinking reagents and conditions, for both collagen crosslinking and covalent linking of the binding agent to the collagen, are known to those of ordinary skill in the art. For example, when using a succinimidyl active acid ester

crosslinking agent the collagen-binding agent composite is immersed in a solution of the crosslinking agent typically at a concentration from about 0.1 to about 15.0% (w/v) and maintained at a temperature from about 2° to 40°C for a period of time of about 1 to 96 hours. Alternatively and preferably, crosslinking may be performed by dehydration of the intermediate collagen involves heating from about 50° to 200°C typically in a vacuum of about 6.67 Pa (50 millitorr) or less for about 1 to 96 hours (so-called "dehydro thermal crosslinking" method).

Alternatively, the crosslinking agent may be premixed with the collagen and/or binding agent prior to drying.

It will be realized that the degree of crosslinking (i.e., controlled by the conditions and/or the amount of crosslinking reagent), may be readily determined by those of ordinary skill in the art to attain the uptake and/or release characteristics of the particular drug which are desired from the collagen composite. Furthermore, the amount of crosslinking may be adjusted to limit to an acceptable minimum the amount of the binding agent which may be leached from the composite during use in the body fluids. It is intended that the crosslinking will prevent the binding agent from being released from the composite so that the primary substance being released from the composite into the body fluid will be the bioactive agent, the release characteristics of which are tailored by the ionic, hydrophobic and/or hydrophilic interaction with the binding agent. Moreover, since the binding agent is essentially retained within the composite, the composite may be reloaded in situ with the bioactive agent. For example, a composite which is a corneal shield may be reloaded by use of liquid drops containing the bioactive agent. The bioactive agent, or at least some of it, will be absorbed into and adsorbed onto the shield, then slowly released over time. This reloading of the composite may continue in situ until the composite is dissolved by the bodily fluids.

A particular advantage of the present invention is that the binding agent will increase the bound fraction of the bioactive agent in the collagen composite as compared to the bound fraction of the bioactive agent in collagen not modified with the binding agent. In a given sample of collagen composite (or other absorbent material) immersed into and in equilibrium with a fluid containing the bioactive agent, the amount of bioactive agent in the composite in excess of the amount due to passive fluid absorption is considered the bound portion. The percentage of the bioactive agent which is bound ($K_b$ x 100) can be calculated as shown in the examples.

The bioactive agents which may be utilized according to the present invention include glaucoma treatment agents such as beta-blockers, spinephrine, carbonic anhydrase inhibitors, prostaglandin derivatives and pilocarpine, gentamicin (a cationic drug), tobramycin, amikacin, butirosin, didesoxykanamycin, fortimycin, kanamycin, lividomycin, neomycin, netilmycin, chloramphenicol and its derivatives such as thiamphenicol, erythromycins, lactone antibiotics such as novobiocin, leucomycins such as spiramycin, lincomycins such as clindamycin; anesthetics; macrolides such as rosamycin; quinolones; penicillins such as ampicillin; peptide antibiotics such as bacitracin, rifamycins; heparin; steroids such an dexamethasone, streptomycins, tetracyclones such as tetracycline, cephalosporins, as well as other antibiotics such as cycloserine, Preferred antibiotics are tobramycin, gentamicin and quinolones. It is also contemplated that combinations or two or more of the bioactive agents may be utilized, such as combinations including anti-inflammatory agents, wound healing promoters other desirable agents which may be distributed at the target site in the body and which may be bound to the collagen composite. In order to bind combinations of bioactive agents, it may be necessary to incorporate combinations of binding agents into the collagen.

The bioactive agent is preferably absorbed by soaking the collagen-binding agent composite in a solution containing the bioactive agent, drying and rehydrating before time of use in the eye (or other body tissue) or rehydrating at the time of use in a solution containing the bioactive agent. It will be realized that the amount of bioactive agent which will be loaded onto the composite will depend upon the amount of binding agent which is present (i.e., ionic hydrophobic and/or hydrophilic attraction of the binding agent to the bioactive agent), upon the concentration of the bioactive agent in the solution into which the composite is soaked, as well as the length of time in which the composite remains in such solution. It is within the skill of those of ordinary skill in the art to determine the proper amount of loading depending upon the release profile and instantaneous dosage which is desired for the drug at the site of the body tissue.

The composite may be in any convenient form for use in or near a body tissue or cavity, such as a film, microspheres, monolithic shapes, sponges, etc. Corneal shield films and microsphere suspensions are particularly useful.

EXAMPLE 1

To illustrate the invention, collagen corneal shields were made containing, in the first case, sodium alginate as an anionic binding agent and, in a second case quaternized cellulose. An unmodified collagen corneal shield was used as a control. The samples were sequentially loaded and eluted with sodium fluorescein (a model for an anionic drug).

Referring to Fig. 1, results are shown of the above-described test with the abscissa showing the number of times the shields were loaded and eluted, and the ordinate showing the ratio of the drug eluted to the total amount of drug which absorbed in the loading stage, normalized to the control (unmodified collagen). After loading the shields, the sodium fluorescein was effluxed or allowed to release into physiological solution and the shield was then reloaded with a drug containing solution. Referring to Fig. 1, the drug loading and release shows a substantial enhancement of drug binding with the cationic quaternary compound (the drug is an anionic compound) and the drug repulsion effect with the

anionic sodium alginate. Sequential drug loading and release shows that the polymeric ionic additive is effective in maintaining the drug affinity over several loading and release cycles although a slight loss of 13% is seen at the third cycle. This demonstrates, for example, that the ionic binding agent has essentially been permanently incorporated into the collagen and allows the corneal shield to be recharged with the drug in situ, for example by the use of the drug in eye drop form.

Referring to Table 1, collagen films containing various amounts of binding agents (sodium alginate, quaternized cellulose, chitosan, or carboxymethylcellulose) were hydrated in drug containing solutions and their fluid swell values calculated to assess drug concentration as a function of fluid volume absorption. The films were then eluted into a tear buffer and the total drug concentration measured.

To calculate the fraction of drug bound to the material, the total drug incorporated is corrected for the passively absorbed drug due to fluid uptake.

$$\text{Total Drug} = \text{Bound Fraction} + \text{Free Fraction}$$

$$1 = \frac{\text{Bound Fraction}}{\text{Total Drug}} + \frac{\text{Free Fraction}}{\text{Total Drug}}$$

$$1 = K_b + K_f$$

Since Kf = Fluid absorbed by material x Drug concentration of surrounding fluid , Kb can be readily calculated from the equilibrium binding results.

TABLE 1

| Collagen Additive | Description | Kb × 100 Gentamicin, @ 4 wt% | Kb × 100 Penicillin @ 10 wt% | Kb × 100 Fluorescein, @ 5 Mm | Kb × 100 Fluorescein, @ 1 Mm |
|---|---|---|---|---|---|
| None | Collagen Alone | 22.5% | 21.3% | 61.7% | 79.5% |
| 10% Alginate | Polyanion | 73.2% | 29.5% | 42.0% | |
| 20% Alginate | Polyanion | 71.5% | | 24.9% | |
| 10% Quat Cellulose | Polycation | 14.8% | 48.8% | 71.6% | |
| 20% Quat Cellulose | Polycation | 6.6% | | 79.9% | |
| 20% Chitosan | Polycation | | | | 89.9% |
| 20% CarboxyMethyl Cellulose | Polyanion | 34.9% | | | |

It is evident from the results that cationic drug gentamicin has a low binding affinity for collagen alone, but the binding attraction is increased with the addition of polyanions and decreased with the addition of polycations. Penicillin, an anionic drug, also demonstrates low binding affinity for collagen, and remains almost unchanged with the addition of polyanions, but increased significantly with the addition of polycation additive. Fluorescein, an anionic diagnostic dye, shows strong natural binding attraction to collagen alone. However even in this case, the binding affinity is increased with the addition of cationic polymeric additives and reduced with the addition of anionic additives.

Referring to Fig. 2, there is shown a graph of three sets of films: a collagen loaded with 2.0% by weight sodium alginate, 10% by weight sodium alginate, and a control collagen. All of these were loaded with gentamicin and the release profile tested for four hours. As shown in Fig. 2, in similarly treated films, the sodium alginate-loaded collagen delivered more drug during the first two hours after exposure to the tear buffer than did the control. Subsequent to two hours, the release rate in all films tended to level off rather uniformly.

Referring to Figs. 3 and 4, are shown the release rates of sodium fluorescein (Fig. 3) and gentamicin (Fig. 4) from collagen films containing a binding agent sodium alginate or quaternized cellulose. Referring to Fig. 3, it can be seen that in the composite containing anionic drug (sodium fluorescein) and cationic binding agent (quaternized cellulose) more drug was released than from the film containing the anionic binding agent sodium alginate. Referring to Fig. 4, it can be seen that the release of the cationic drug gentamicin is enhanced in the collagen bearing the anionic binding, agent (sodium alginate), whereas release is depressed in the collagen bearing the cationic binding agent, quaternized

cellulose.

EXAMPLE 2

Collagen slurries were prepared in deionized water at between 1.25% and 2.0% solids and Ph of 8.0+/-0.2. An appropriated volume of 2% polyethyleneglycol-cholesteryl sebacate (PEG-C) solution was stirred into this mixture to yield a predetermined ratio of collagen to PEG-C in the final dried film. Solution was cast into hemispherical polypropylene molds with a base curve of 8.6 mm and dried under controlled conditions. The dried films were crosslinked under heated vacuum (dehydrothermal crosslinking) for an appropriate amount of time to yield the desired hydration of the finished product. This product is referred to hereafter as a (corneal) shield. After drying and dehydrothermal crosslinking, the collagen composite swells up to 1000% but does not release PEG-C when soaked in aqueous buffers at room temperature.

Dry shields (ten) were weighed and placed into saturated steroid solutions in tear buffer. Drug uptake by the shield did not change after the first few minutes of soaking, indicating that uptake was no slower than the hydration of the shield. Thus shields were soaked for a convenient length of time, typically one to two hours. The wet shields were weighed and transferred into clean dry vials. A known amount of trypsin/buffer solution was added and the tubes were incubated at 37°C for two to four hours or until the shields appeared completely dissolved by visual inspection. This solution was assayed for dexamethasone using HPLC and UV detection. From the dry and wet weights of the shield and the soaking solution concentration, the amount of drug passively soaked up by the shield could be determined. The remainder of the assayed drug was assumed to be bound in some manner to the composite matrix. The uptake of shields ranging from 0 to 20% POE-C was determined.

Since the volume of the tear film is 7-10 μl, and the volume of the hydrated shield is typically 25 μl, an exact model of the delivery of drug to the tear film from a corneal shield was not possible. A small volume cell was machined which would allow good mixing between inlet solution and solution in the cell. A sample cell was loaded with a pre-soaked corneal shield. Buffer was pumped into the sample cell through two inlets at right angles to each other to afford good mixing of the cell contents. Eluted solution was continuously pumped through the injection loop of an HPLC autosampler system (Spectra-Physics SP8780). At predetermined times, an injection onto a C18 column was made from the contents of the injection loop. By injecting the eluate directly onto a column, the complication of small volume sample collection were overcome. Standard peak analysis was performed, and a plot of elution concentration versus time was generated for both 100% collagen and 20% PEG-C composite collagen shields.

Figure 5A shows a plot of total dexamethasone uptake for shields of various composition soaked in aqueous saturated dexamethasone solutions. The x-axis shows the amount of PEG-C incorporated into the collagen shield, ranging from 0 to 20% incorporation. The solid squares represent the calculated amount of dexamethasone passively soaked up by the shields due to swelling. The open squares show the total amount of drug taken up by the shields. The difference between total uptake and uptake due to swell normalized to total drug content is the bound drug fraction, $K_b$. It is most apparent from this graph that the addition of increasing amounts of PEG-C to the collagen shield greatly increases the amount of dexamethasone bound by the shield. This is more clearly illustrated in Fig. 5B which shows the increase in $K_b$ for dexamethasone as related to the amount of PEG-C in the composite collagen material. Figure 6 is a plot summary of a similar uptake experiment run with the steroidal compound cortisone in the soaking solutions. Again, the same behavior of increased drug binding at higher weight fractions of PEG-C in the shield is shown. Figures 7 and 8 show elution profiles for the 100% collagen and 20% PEG-C shields. Figure 7 is a linear y-axis plot which shows that dexamethasone is released at much higher concentrations throughout the release experiment. The area under the PEG-C release curve is roughly four time the area under the 100% collagen release curve, which is in agreement with the drug uptake results. Figure 8A is a log y-axis plot of the same data shown in Figure 7. From this plot it is apparent that not only is the dexamethasone releasing at a higher concentration from the modified shield, but that it is also releasing at a slower rate. The extended half-life of drug release due to the binding additive is illustrated in Fig. 8B, showing the increase in drug half-life as related to PEG-C additive levels.

The data presented above demonstrate a novel method of enhancing and sustaining uptake and release of steroidal drugs from collagen hydrogels.

## Claims

1. A collagen-based control-release delivery system for use in contact with body fluid or tissue comprising:

   (a) a composition comprising a binding agent which has been incorporated into collagen by contacting said binding agent with said collagen and then crosslinking said collagen or by covalently linking said binding agent to said collagen; and
   (b) a pharmacologically active material absorbed into said composition, whereby the amount and/or strength

of binding of said active material in said collagen is altered by ionic, hydrophobic, and/or hydrophilic interaction between said binding agent and said active material to enhance or attenuate the rate of and/or amount of release of said material from said collagen into said body fluid or tissue.

2. A system according to Claim 1 characterized by a bound fraction, $K_b$, of said pharmacologically active material greater than the bound fraction of said pharmacologically active material measured in similar collagen not incorporating said binding agent.

3. A system according to any preceding Claim wherein said binding agent is selected from alginate, quaternized cellulose, chitosan, polyacrylic acid-containing compounds, polyalkyleneglycol-containing compounds and carboxymethylcellulose.

4. A system according to Claim 3 wherein said binding agent is a polyalkylene-glycol-containing compound selected from polyethyleneglycol- and polypropylene-glycol-containing compounds.

5. A system according to any preceding claim wherein said pharmacologically active material comprises an antibiotic.

6. A system according to any of Claims 1 to 4 wherein said active material comprises a growth factor.

7. A system according to any of Claims 1 to 4 wherein said active material comprises a steroid.

8. A system according to any of Claims 1 to 4 wherein said active material comprises an agent for glaucoma treatment.

9. A system according to any preceding claim wherein said collagen is crosslinked by thermal dehydration.

10. A system according to any of Claims 1 to 8 wherein said collagen is crosslinked by chemical crosslinking agents.

11. A system according to any preceding Claim in the form of a corneal shield.

12. A system according to any of claims 1 to 10 in the form of microspheres.

13. A system according to any of claims 1 to 10 in the form of an ocular insert.

14. A system according to any of Claims 5 and 9 to 13 wherein said active material comprises tobramycin.

15. A system according to any of Claims 5 and 9 to 14 wherein said active material further comprises dexamethasone.

16. A system according to any preceding Claim wherein said binding agent comprises a polyalkyleneglycol-modified drug analogue.

17. A system according to Claim 16 wherein said drug analogue comprises a steroid.

18. A system according to Claim 16 wherein said modified drug analogue comprises polyethyleneglycol-cholesteryl sebacate.

19. A system according to Claim 18 wherein said pharmacologically active material comprises a steroid.

20. A system according to Claim 18 wherein said pharmacologically active material comprises dexamethasone.

21. A system according to Claim 18 wherein said pharmacologically active material comprises cortisone.

22. A system according to any preceding Claim comprising two or more different binding agents.

23. A system according to any preceding Claim comprising two or more different pharmacologically active materials.

## Patentansprüche

1. Zuführsystem auf Collagenbasis für kontrollierte Freisetzung zur Verwendung in Kontakt mit Körperflüssigkeit oder Gewebe, umfassend:

   a) eine ein Bindemittel enthaltende Zusammensetzung, die in Collagen inkorporiert wurde durch Kontaktierung des Bindungsmittels mit dem Collagen und anschließendes Vernetzen des Collagens oder durch kovalente Bindung des Bindemittels an das Collagen; und
   b) einen in der Zusammensetzung absorbierten pharmakologischen Wirkstoff, wobei die Menge und/oder Bindungsstärke des Wirkstoffs in dem Collagen geändert wird durch ionische, hydrophobe und/oder hydrophile Wechselwirkung zwischen dem Bindemittel und dem Wirkstoff zur Verstärkung oder Schwächung der Freisetzungsrate und/oder -menge des Wirkstoffs von dem Collagen in die Körperflüssigkeit oder das Gewebe.

2. System nach Anspruch 1,
   **gekennzeichnet** durch eine gebundene Fraktion $K_b$ des pharmakologischen Wirkstoffs, die größer ist als die gebundene Fraktion des pharmakologischen Wirkstoffs, die in ähnlichem Collagen, das das Bindemittel nicht enthält, gemessen wird.

3. System nach einem vorangehenden Anspruch,
   bei dem das Bindemittel ausgewählt ist aus Alginat, quaternisierter Zellulose, Chitosan, Polyacrylsäure-enthaltenden Verbindungen, Polyalkylglykol-enthaltenden Verbindungen und Carboxymethylcellulose.

4. System nach Anspruch 3,
   bei dem das Bindemittel eine Polyalkylenglykol enthaltende Verbindung ist, die ausgewählt ist aus Polyethylenglycol- und Polypropylenglycol-enthaltenden Verbindungen.

5. System nach einem vorangehenden Anspruch,
   bei dem der pharmakologische Wirkstoff ein Antibiotikum umfaßt.

6. System nach einem der Ansprüche 1 bis 4,
   bei dem der Wirkstoff einen Wachstumsfaktor umfaßt.

7. System nach einem der Ansprüche 1 bis 4,
   bei dem der Wirkstoff ein Steroid umfaßt.

8. System nach einem der Ansprüche 1 bis 4,
   bei dem der Wirkstoff einen Wirkstoff zur Behandlung von Glaukom umfaßt.

9. System nach einem vorangehenden Anspruch,
   bei dem das Collagen durch thermische Dehydratisierung vernetzt ist.

10. System nach einem der Ansprüche 1 bis 8,
    bei dem das Collagen durch chemische Vernetzungsmittel vernetzt ist.

11. System nach jedem vorangehenden Anspruch,
    in der Form eines Hornhautschildes.

12. System nach einem der Ansprüche 1 bis 10,
    in der Form von Mikrokugeln.

13. System nach einem der Ansprüche 1 bis 10,
    in der Form eines Augeneinsatzes.

14. System nach einem der Ansprüche 5 und 9 bis 13,
    bei dem der Wirkstoff Tobramycin umfaßt.

15. System nach einem der Ansprüche 5 und 9 bis 14,
    bei dem der Wirkstoff zusätzlich Dexamethason umfaßt.

**16.** System nach jedem vorangehenden Anspruch, bei dem das Bindemittel ein mit Polyalkylenglykol modifiziertes Arneimittel-Analogon umfaßt.

**17.** System nach Anspruch 16, bei dem das Arzneimittel-Analogon ein Steroid umfaßt.

**18.** System nach Anspruch 16, bei dem das modifizierte Arzneimittel-Analogon Polyethylenglykol-Cholesteryl-Sebacat umfaßt.

**19.** System nach Anspruch 18, bei dem der pharmakologische Wirkstoff ein Steroid umfaßt.

**20.** System nach Anspruch 18, bei dem der pharmakologische Wirkstoff Dexamethason umfaßt.

**21.** System nach Anspruch 18, bei dem der pharmakologische Wirkstoff Cortison umfaßt.

**22.** System nach jedem vorangehenden Anspruch, welches zwei oder mehr unterschiedliche Bindemittel enthält.

**23.** System nach jedem vorangehenden Anspruch, das zwei oder mehr unterschiedliche pharmakologische Wirkstoffe enthält.

## Revendications

**1.** Un système de délivrance à libération contrôlée à base de collagène pour servir en contact avec un fluide ou tissu corporel comprenant :

(a) une composition comprenant un agent de liaison qui a été incorporé dans le collagène par mise en contact dudit agent de liaison avec ledit collagène puis par réticulation dudit collagène ou par liaison covanlente dudit agent de liaison audit collagène; et
(b) un produit pharmacologiquement actif absorbé dans ladite composition, de sorte que la quantité et/ou la force de liaison dudit produit actif dans ledit collagène est modifiée par interaction ionique, hydrophobe et/ou hydrophile entre ledit agent de liaison et ledit produit actif pour augmenter ou atténuer la vitesse et/ou la quantité libérée dudit produit a partir dudit collagène dans lesdits fluide ou tissu corporels.

**2.** Un système selon la revendication 1, caractérisé en ce que la fraction liée, $K_b$, dudit produit pharmacologiquement actif est supérieure à la fraction liée dudit produit pharmacologiquement actif mesuré dans un collagène similaire ne contenant pas ledit agent de liaison.

**3.** Un système selon une quelconque des revendications précédentes dans lequel ledit agent de liaison est choisi parmi alginate, cellulose quaternizée, chitosane, composés renfermant de l'acide polyacrylique, composé contenant du polyalkylèneglycol et carboxyméthylcellulose.

**4.** Un système selon la revendication 3 dans lequel ledit agent de liaison est un composé contenant un polyalkylèneglycol choisi parmi les composés contenant du polyéthylène-glycol et du polypropylène-glycol.

**5.** Un système selon une quelconque des revendications précédentes dans lequel ledit produit pharmacologiquement actif comprend un antibiotique.

**6.** Un système selon une quelconque des revendications 1 à 4 dans lequel ledit produit actif comprend un facteur de croissance.

**7.** Un système selon une quelconque des revendications 1 à 4 dans lequel le produit actif comprend un stéroïde.

**8.** Un système selon une quelconque des revendications 1 a 4 dans lequel ledit produit actif comprend un agent de traitement du glaucome.

9. Un système selon une quelconque des revendications précédentes dans lequel ledit collagène est réticulé par déshydratation thermique.

10. Un système selon une quelconque des revendications 1 à 8 dans lequel ledit collagène est réticulé par des agents de réticulation chimiques.

11. Un système selon une quelconque des revendications précédentes sous la forme d'une protection cornéenne.

12. Un système selon une quelconque des revendications 1 à 10 sous la forme de microsphères.

13. Un système selon une quelconque des revendications 1 à 10 sous la forme d'un implant oculaire.

14. Un système selon une quelconque des revendications 5 et 9 à 13 dans lequel ledit produit actif comprend la tobramycine.

15. Un système selon une quelconque des revendications 5 et 9 à 14 dans lequel ledit produit actif comprend en outre la dexaméthasone.

16. Un système selon une quelconque des revendications précédentes dans lequel ledit agent de liaison comprend un analogue de médicament modifié par du polyalkylèneglycol.

17. Un système selon la revendication 16 dans lequel ledit analogue de médicament comprend un stéroïde.

18. Un système selon la revendication 16 dans lequel ledit analogue de médicament modifié comprend un polyéthylèneglycol-cholestéryl sébaçate.

19. Un système selon la revendication 18 dans lequel ledit produit pharmacologiquement actif comprend un stéroïde.

20. Un système selon la revendication 18 dans lequel ledit produit pharmacologiquement actif comprend la dexaméthasone.

21. Un système selon la revendication 18 dans lequel ledit produit pharmacologiquement actif comprend la cortisone.

22. Un système selon une quelconque des revendications précédentes comprenant au moins deux agents de liaison.

23. Un système selon une quelconque des revendications précédentes comprenant au moins deux produits pharmacologiquement actifs différents.

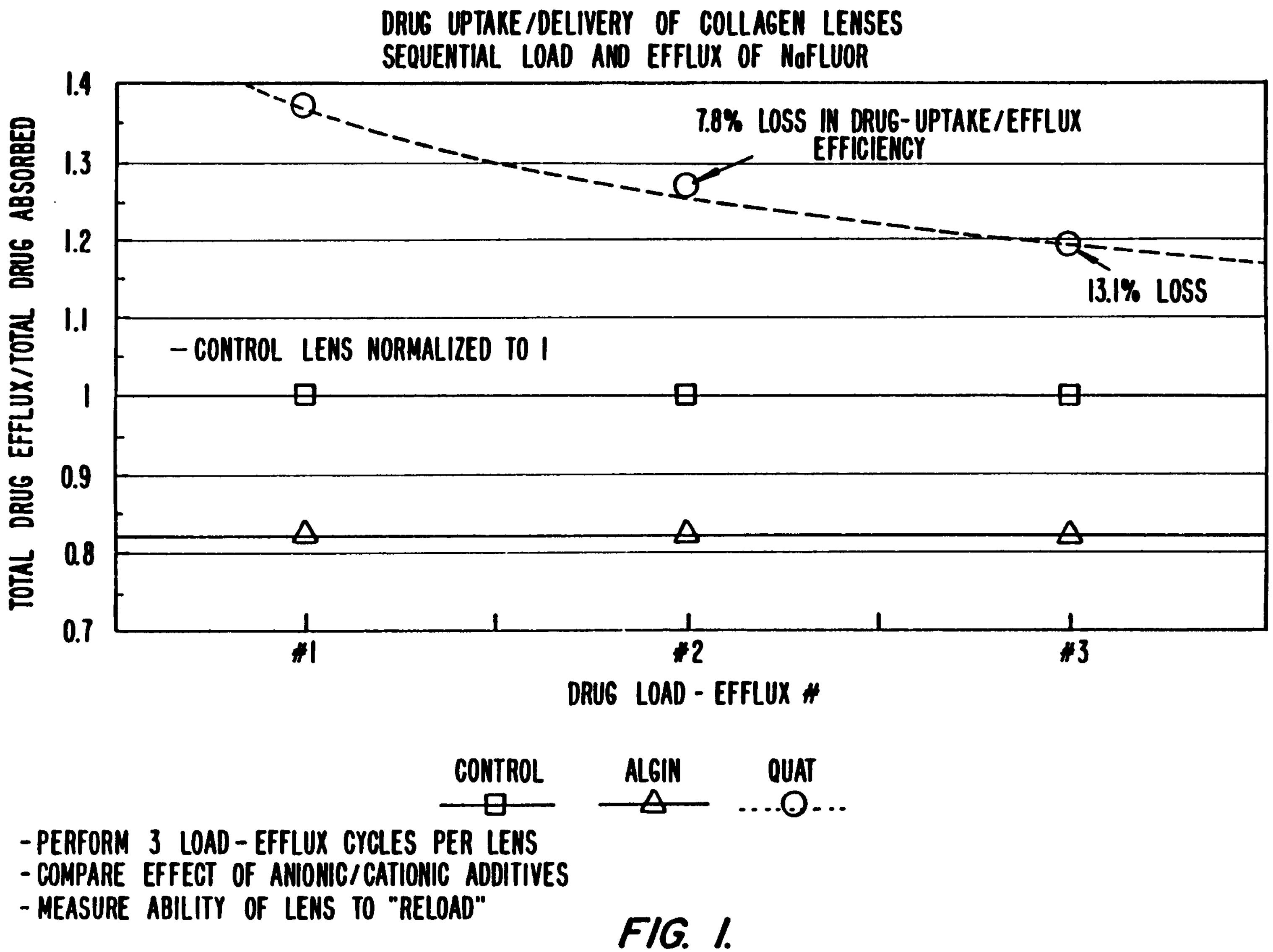
DRUG UPTAKE/DELIVERY OF COLLAGEN LENSES
SEQUENTIAL LOAD AND EFFLUX OF NaFLUOR
TOTAL DRUG EFFLUX/TOTAL DRUG ABSORBED
1.4
1.3
1.2
1.1
1
0.9
0.8
0.7
7.8% LOSS IN DRUG-UPTAKE/EFFLUX EFFICIENCY
13.1% LOSS
- CONTROL LENS NORMALIZED TO 1
#1
#2
#3
DRUG LOAD - EFFLUX #
CONTROL
ALGIN
QUAT
- PERFORM 3 LOAD - EFFLUX CYCLES PER LENS
- COMPARE EFFECT OF ANIONIC/CATIONIC ADDITIVES
- MEASURE ABILITY OF LENS TO "RELOAD"

FIG. 1.

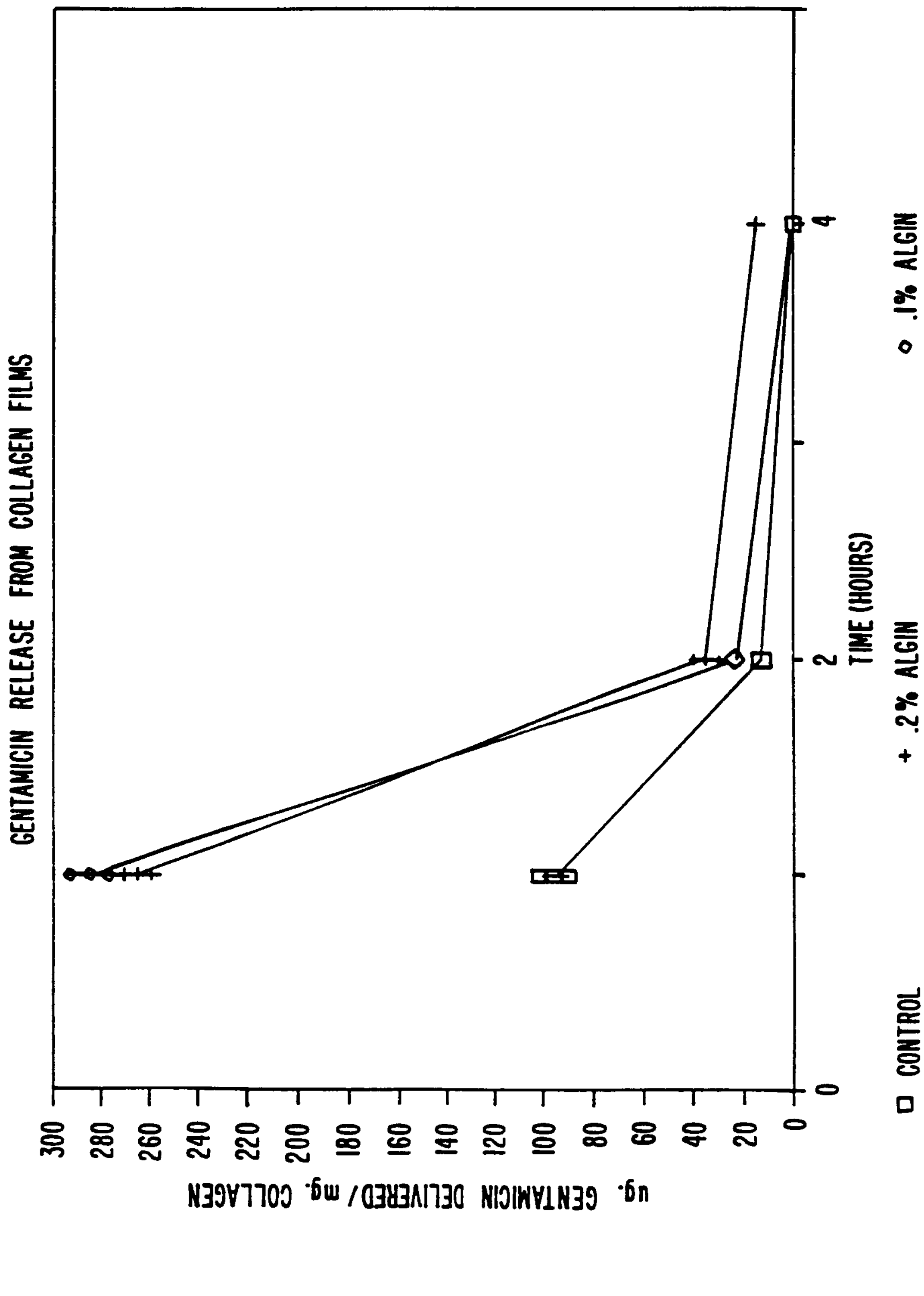
GENTAMICIN RELEASE FROM COLLAGEN FILMS
ug. GENTAMICIN DELIVERED/ mg. COLLAGEN
300
280
260
240
220
200
180
160
140
120
100
80
60
40
20
0
0
2
4
TIME (HOURS)
□ CONTROL
+ .2% ALGIN
◇ .1% ALGIN

FIG. 2.

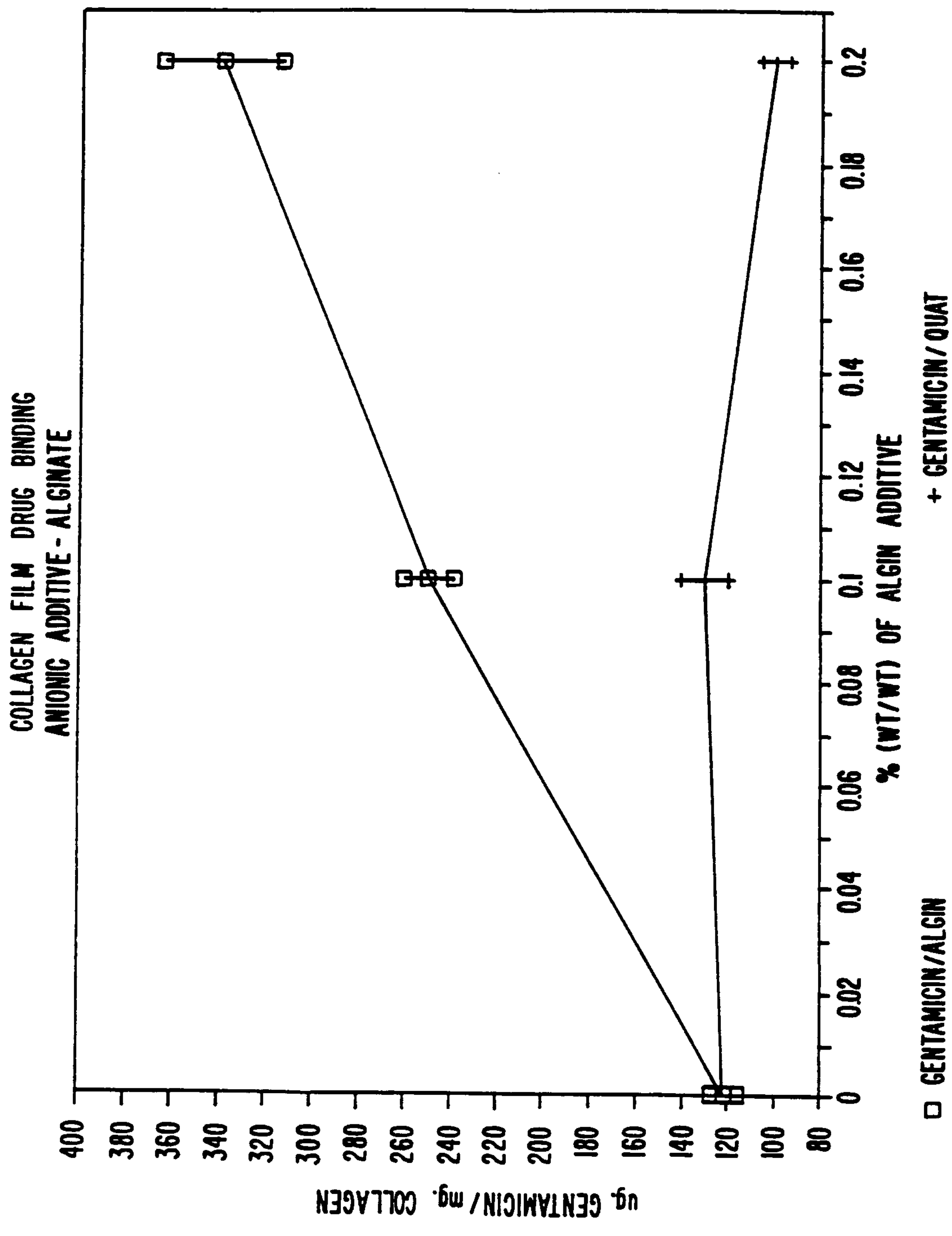
COLLAGEN FILM DRUG BINDING
ANIONIC ADDITIVE - ALGINATE
ug. GENTAMICIN / mg. COLLAGEN
400
380
360
340
320
300
280
260
240
220
200
180
160
140
120
100
80
0
0.02
0.04
0.06
0.08
0.1
0.12
0.14
0.16
0.18
0.2
% (WT/WT) OF ALGIN ADDITIVE
□ GENTAMICIN/ALGIN
+ GENTAMICIN/QUAT

FIG. 3.

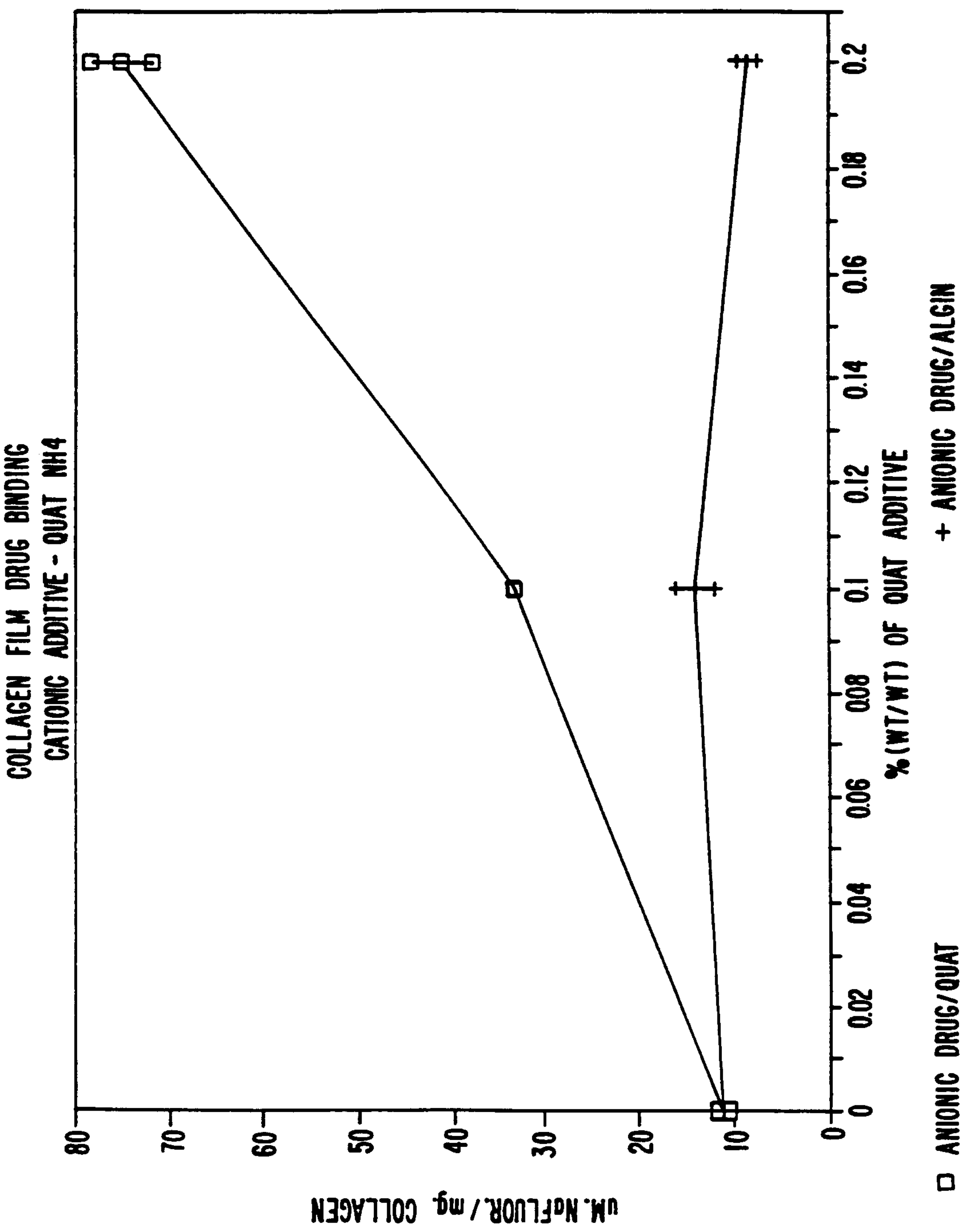
COLLAGEN FILM DRUG BINDING
CATIONIC ADDITIVE - QUAT NH4
uM. NaFLUOR. / mg. COLLAGEN
80
70
60
50
40
30
20
10
0
0
0.02
0.04
0.06
0.08
0.1
0.12
0.14
0.16
0.18
0.2
% (WT/WT) OF QUAT ADDITIVE
□ ANIONIC DRUG/QUAT
+ ANIONIC DRUG/ALGIN

FIG. 4.

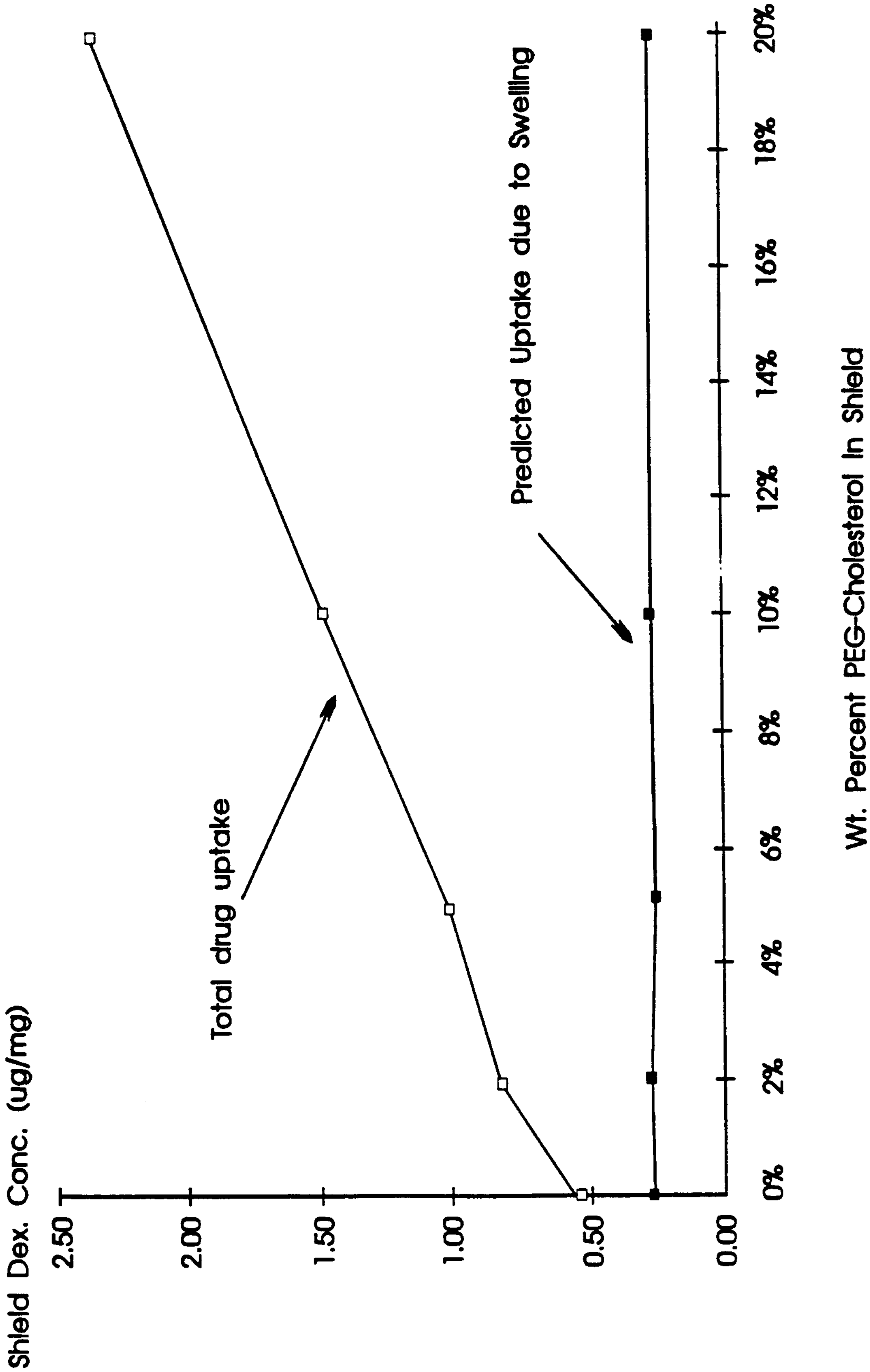
Shield Dex. Conc. (ug/mg)
2.50
2.00
1.50
1.00
0.50
0.00
Total drug uptake
Predicted Uptake due to Swelling
0%
2%
4%
6%
8%
10%
12%
14%
16%
18%
20%
Wt. Percent PEG-Cholesterol In Shield

FIG. 5A

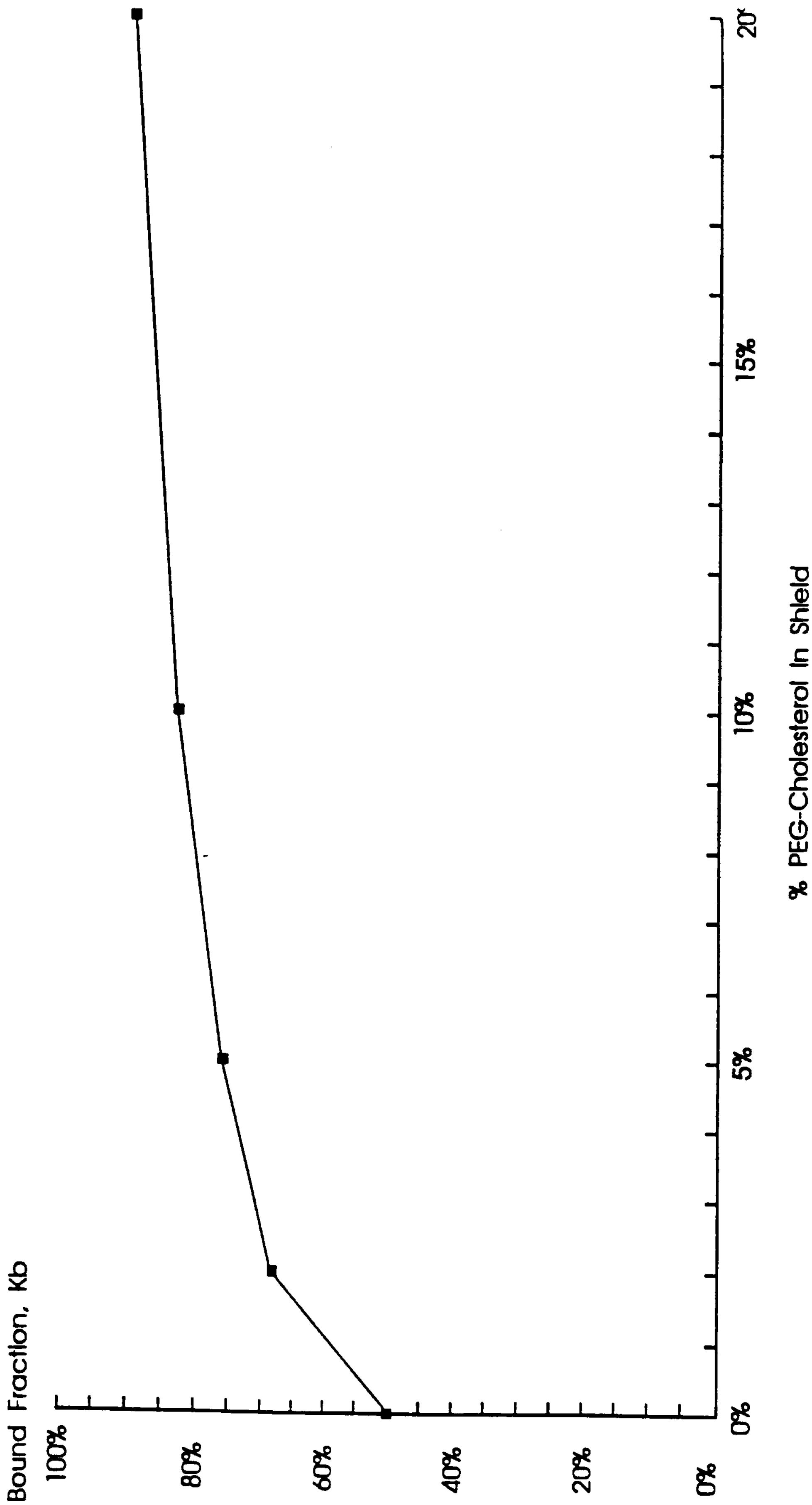
Bound Fraction, Kb
100%
80%
60%
40%
20%
0%
0%
5%
10%
15%
20%
% PEG-Cholesterol In Shield

FIG 5B

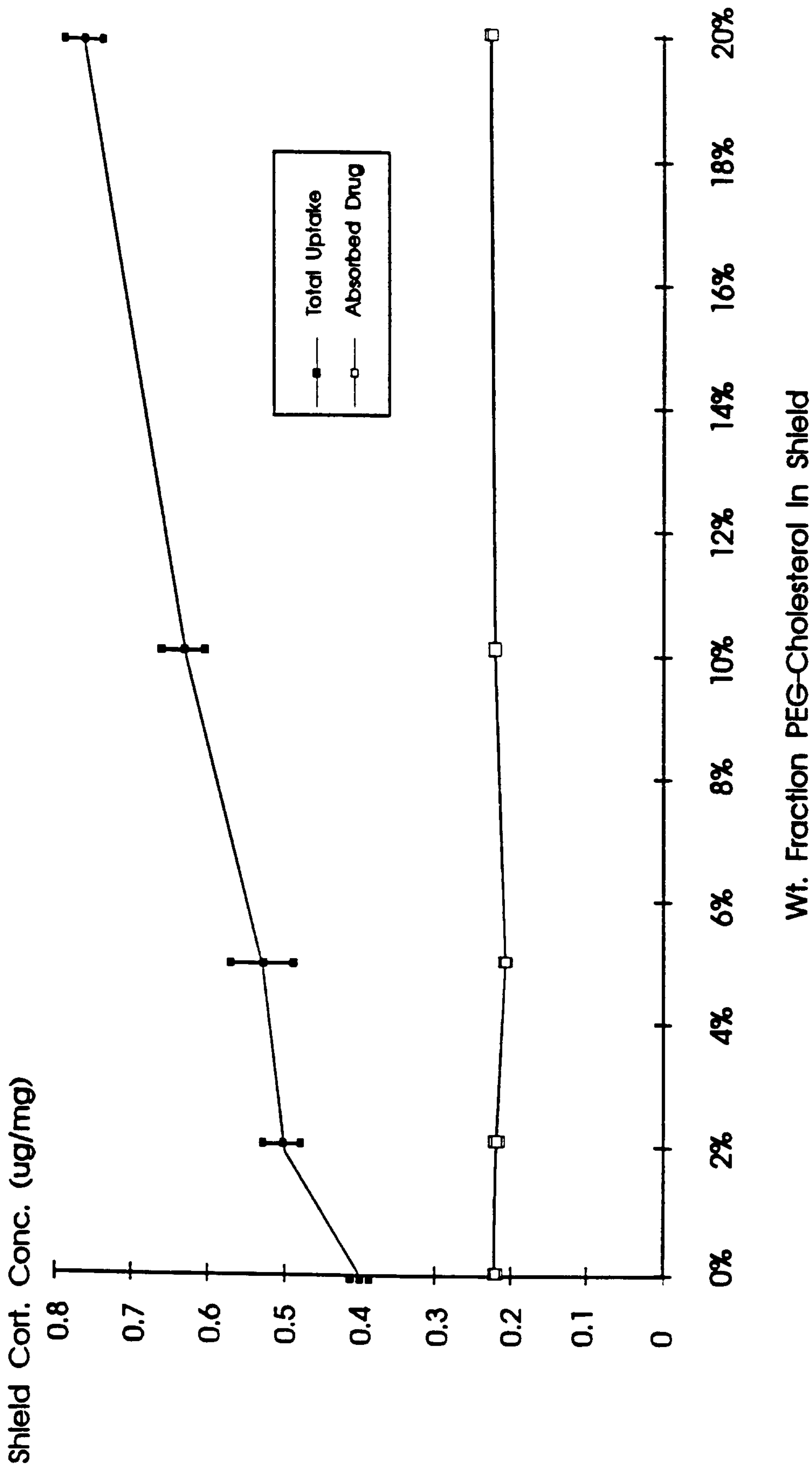
Shield Cort. Conc. (ug/mg)
0.8
0.7
0.6
0.5
0.4
0.3
0.2
0.1
0
Total Uptake
Absorbed Drug
0%
2%
4%
6%
8%
10%
12%
14%
16%
18%
20%
Wt. Fraction PEG-Cholesterol In Shield

FIG. 6

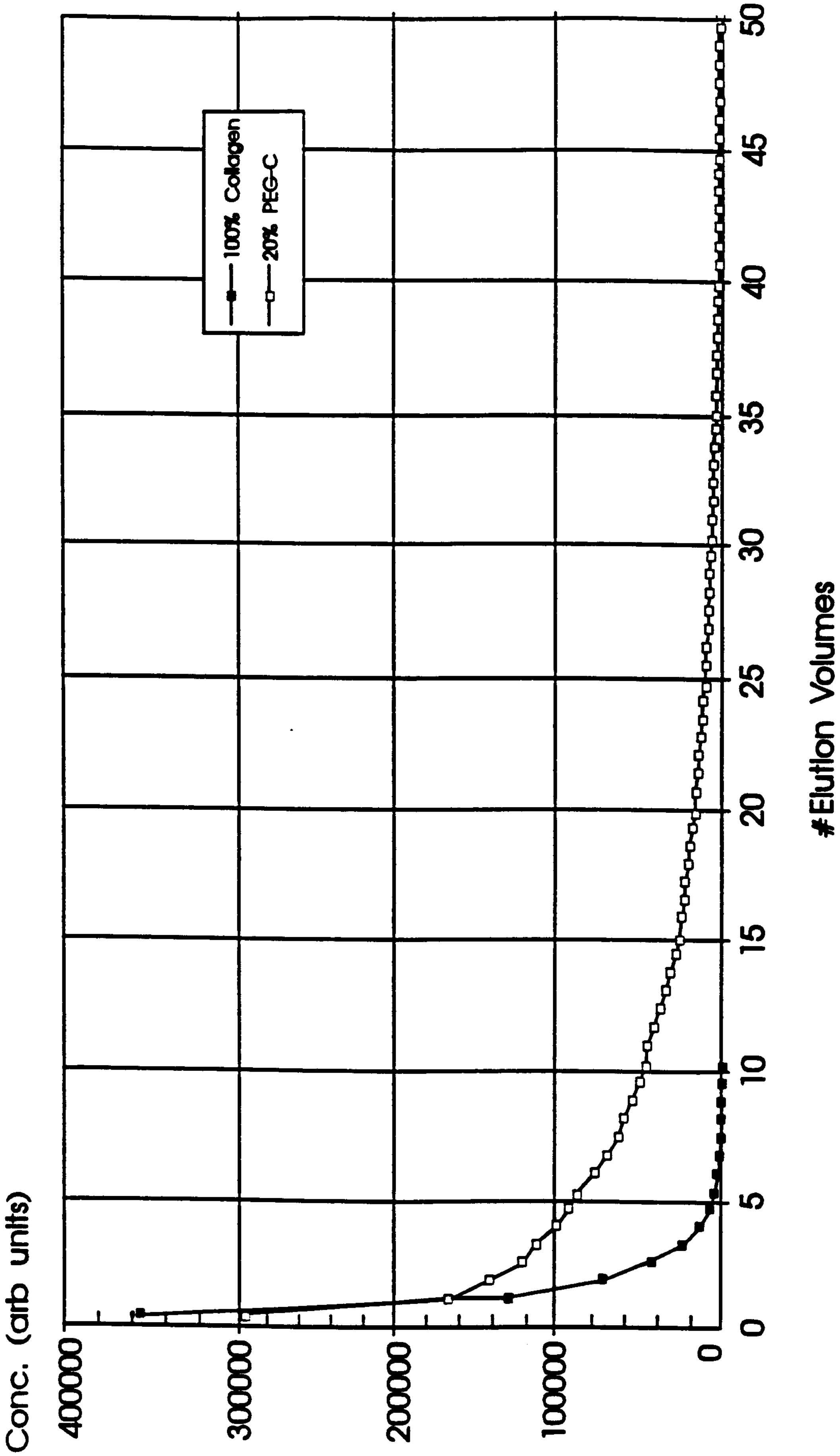
Conc. (arb units)
400000
300000
200000
100000
0
0
5
10
15
20
25
30
35
40
45
50
100% Collagen
20% PEG-C
#Elution Volumes

FIG. 7

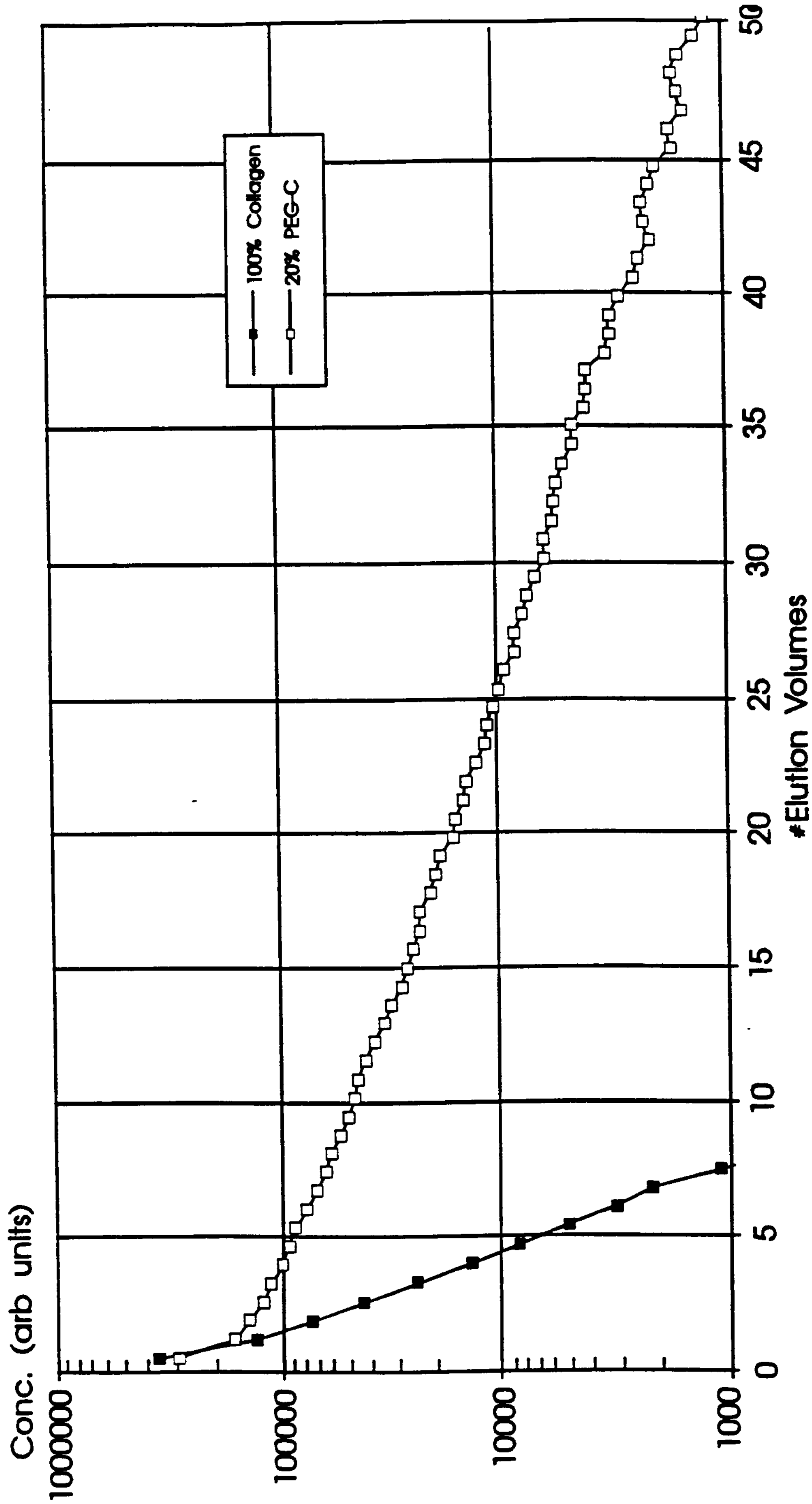
Conc. (arb units)
1000000
100000
10000
1000
100% Collagen
20% PEG-C
0
5
10
15
20
25
30
35
40
45
50
#Elution Volumes

FIG. 8A

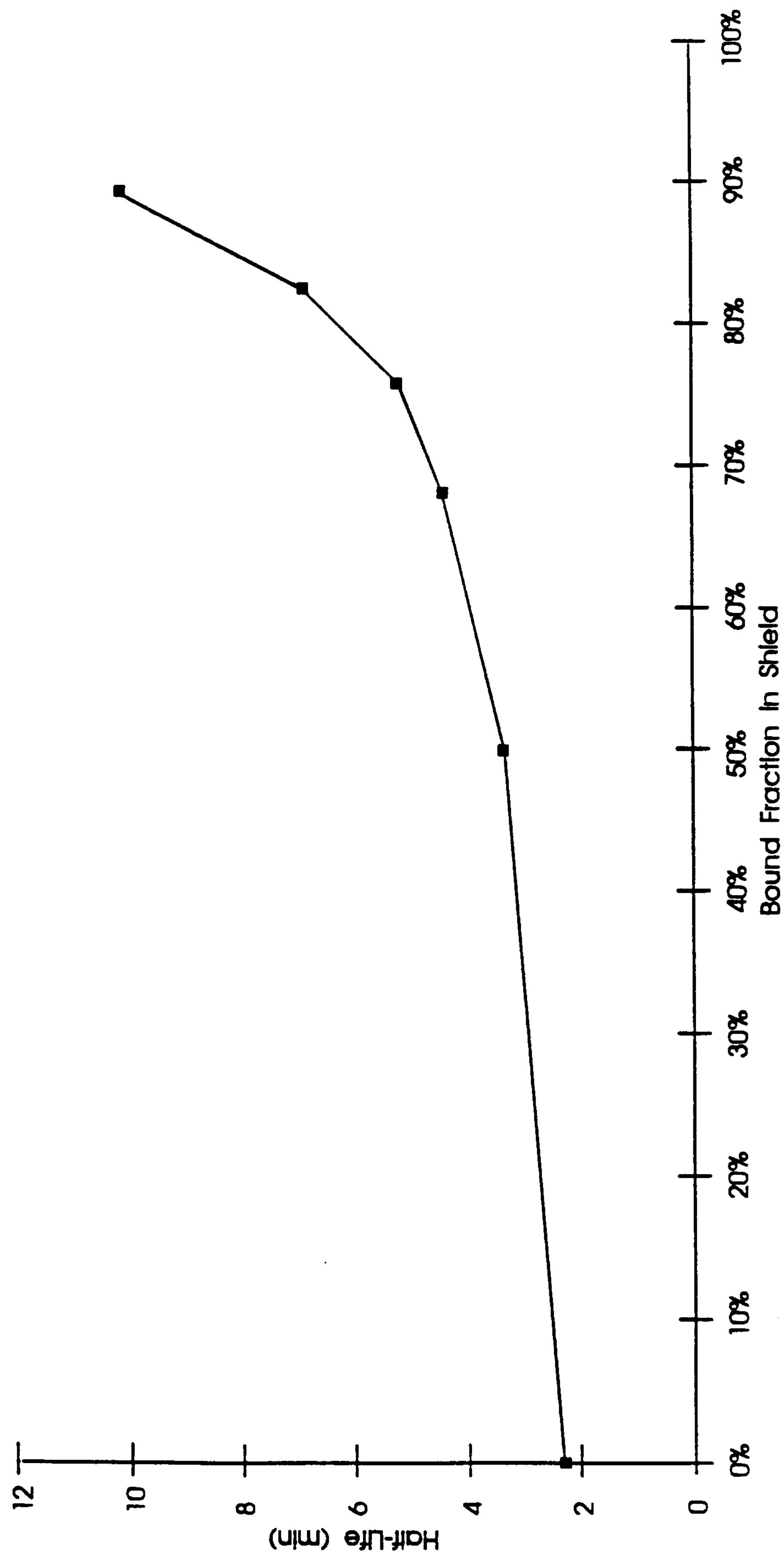
Half-Life (min)
0
2
4
6
8
10
12
Bound Fraction In Shield
0%
10%
20%
30%
40%
50%
60%
70%
80%
90%
100%

FIG. 8B